## Europäisches Patentamt

(19) **European Patent Office**

**Office européen des brevets**

(11) Veröffentlichungsnummer: **0 021 330**
**B1**

(12) **EUROPÄISCHE PATENTSCHRIFT**

(45) Veröffentlichungstag der Patentschrift:
25.05.83

(21) Anmeldenummer: 80103378.8

(22) Anmeldetag: 18.06.80

(51) Int. Cl.³: **C 07 C 31/08**, C 07 C 29/15,
B 01 J 23/76, B 01 J 23/84

(54) Verfahren zur Herstellung von Ethanol aus Synthesegas.

(30) Priorität: 25.06.79 DE 2925571

(43) Veröffentlichungstag der Anmeldung:
07.01.81 Patentblatt 81/1

(45) Bekanntmachung des Hinweises auf die Patenterteilung:
25.05.83 Patentblatt 83/21

(84) Benannte Vertragsstaaten:
BE DE FR GB IT NL

(56) Entgegenhaltungen:
FR-A-660 678
US-A-3 947 563
US-A-4 150 246

(73) Patentinhaber: HOECHST AKTIENGESELLSCHAFT,
Postfach 80 03 20, D-6230 Frankfurt/Main 80 (DE)

(72) Erfinder: Hachenberg, Horst, Dr., Mohnweg 1,
D-6229 Walluf (DE)
Erfinder: Wunder, Friedrich, Dr., Jahnstrasse 46,
D-6093 Flörsheim am Main (DE)
Erfinder: Leupold, Ernst Ingo, Dr., Am Zäunefeld 15,
D-6392 Neu-Anspach (DE)
Erfinder: Schmidt, Hans-Joachim, Dr., Am Burgenblick 6,
D-6240 Königstein/Taunus (DE)

## Verfahren zur Herstellung von Ethanol aus Synthesegas

Die vorliegende Erfindung betrifft ein Verfahren zur Herstellung von Ethanol durch Umsetzung von Kohlenmonoxid mit Wasserstoff an einem durch Gold und/oder Silber und/oder Rhenium modifizierten Kobaltkatalysator.

Verfahren zur Herstellung von Alkoholen aus Synthesegas sind bereits bekannt.

So ist bekannt, daß Rhodiumkatalysatoren mit bestimmten Promotoren die Synthesegasreaktion in Richtung sauerstoffhaltige Verbindungen lenken (DE-AS 2 503 204). Der Nachteil liegt im relativ hohen Preis des Rhodiums. Auch ist bekannt, daß billigere Kobalt-Katalysatoren (Fischer Tropsch) bei der Synthesegasreaktion Verwendung finden. Hierbei entsteht jedoch eine Vielzahl von Produkten, vorwiegend Kohlenwasserstoffe und nur geringe Mengen sauerstoffhaltige Verbindungen.

Die Modifizierung von Kobaltkatalysatoren durch Kupfer, Chrom, Zink, Erdalkalimetalle, Alkalimetalle, Aluminium, seltene Erden oder Eisen liefert zwar eine Reihe von Produkten von Methanol bis Butanol, wobei jedoch der Ethanolanteil nur ca. 30 Gew.-% beträgt (DE-PS 857 799, DE-PS 544 665, FR-PS 660 678, FR-PS 1 074 045, FR-PS 2 748 097). Außerdem sind diese Katalysatoren sehr empfindlich, was sowohl die Herstellung, die Inbetriebnahme aus den laufenden Betrieb angeht. Sie sind deshalb meist nicht langlebig. Es bestand somit die Aufgabe, Ethanol aus Synthesegas mit hoher Selektivität an robusten und langlebigen Katalysatoren herzustellen.

Es wurde nun gefunden, daß die Selektivität von Ethanol an Kobaltkatalysatoren durch Zusatz der Promotoren Gold und/oder Silber und/oder Rhenium ganz wesentlich erhöht wird. Gegenstand der Erfindung ist daher ein Verfahren zur Herstellung von Ethanol aus Kohlenmonoxid und Wasserstoff an Kobaltkatalysatoren, dadurch gekennzeichnet, daß der Katalysator außer Kobalt als Metall oder Verbindung noch mindestens eins der Elemente Gold, Silber und Rhenium als Metall oder Verbindung enthält. Vorzugsweise erhält der Katalysator außer Kobalt noch Gold, insbesondere aber die Kombination Gold/Silber. Der Befund, daß die Selektivität der Synthesegasreaktion zu Ethanol durch Zusatz der genannten Elemente so wesentlich erhöht wird — bei gleichzeitig guter Raumzeitleistung — ist außerordentlich überraschend und nicht voraussehbar.

Neben Ethanol, das bei dem erfindungsgemäßen Verfahren in hoher Selektivität entsteht, bilden sich als Nebenprodukte in geringer Menge Methanol, Propanol und Butanol und etwas Acetaldehyd.

Die Katalysatoren für das erfindungsgemäße Verfahren werden vorzugsweise durch Tränken eines Katalysatorträgers mit einer Lösung von Kobalt-, Gold- und/oder Silber- und/oder Rheniumsalzen und anschließendes Trocknen hergestellt, wobei die Salze gemeinsam oder nacheinander aufgebracht werden können. Als Salze eignen sich alle löslichen Kobalt-, Gold-, Silber- und Rheniumsalze z. B. die Chloride, Bromide, Nitrate, Acetate, Propionate, Lactate, Citrate des Kobalts, die Chloride, Acetate, Propionate des Golds, sowie Aurate (wie Bariumacetoaurat oder Bariumpropionoaurat), ferner das Acetat, Propionat, Butyrat, Lactat und Nitrat des Silbers, sowie Rheniumheptoxid, Perrhenate und Rhenate.

Die genannten Salze werden in geeigneten Lösungsmitteln gelöst.

Als Lösungsmittel eignen sich z. B. Wasser, wäßrige Carbonsäuren und wasserfreie Carbonsäuren, insbesondere Wasser und Essigsäure.

Als Träger können die üblichen Trägermaterialien mit unterschiedlichen spezifischen Oberflächen und Porenvolumina verwendet werden. Allerdings werden Träger mit spezifischer Oberfläche von 50 – 1000 m²/g und Porenvolumina über 0,3 ml/g bevorzugt.

Geeignet sind z. B. Kieselsäure, natürliche oder synthetische Silikate von Elementen der II. bis VIII. Gruppe des Periodischen Systems (beispielsweise die Silikate des Magnesiums, Aluminiums oder Mangans), ferner Aluminiumoxid, Thoriumoxid und Spinelle.

Es ist jedoch auch möglich, geeignete Katalysatoren durch Fällung von Kobaltoxid gemeinsam mit Gold und/oder Silber und/oder Rhenium oder durch nachträgliche Imprägnierung von Kobaltoxid mit Salzen von Gold und/oder Rhenium herzustellen.

Vorzugsweise reduziert man den Katalysator vor seinem Einsatz mit einem geeigneten Reduktionsmittel, wie Wasserstoff, Kohlenmonoxid oder Methanol, und zwar vorteilhafterweise im Synthesegas-Reaktor selbst. Im allgemeinen arbeitet man dabei unterhalb von 400° C, vorzugsweise zwischen 200 und 300° C. Vielfach ist es zweckmäßig, die Reduktion nicht mit den unverdünnten reduzierend wirkenden Gasen, sondern mit einem zusätzlichen Anteil von Inertgasen, wie z. B. Stickstoff, Kohlendioxid und Edelgasen durchzuführen.

Die Konzentration des Kobalts in den Katalysatoren kann in sehr weiten Grenzen variiert werden, wobei man trägerfreie Kobaltverbindungen, beispielsweise Kobaltoxid, ebenso verwenden kann, wie geträgerte Katalysatoren mit mindestens 1 Gew.-% Kobalt.

Die Konzentration der Promotoren wählt man in Abhängigkeit von der Kobaltkonzentration und zwar so, daß pro Mol Kobalt 0,001 bis 0,5 Mol, vorzugsweise 0,02 bis 0,2 Mol jedes einzelnen Promotors vorliegen.

Zur Durchführung des erfindungsgemäßen Verfahrens werden Gasgemische, die ganz oder zu einem überwiegenden Teil aus Kohlenmonoxid und Wasserstoff bestehen und daneben gegebenenfalls noch andere Komponenten wie Stickstoff, Argon, Kohlendioxid oder Methan enthalten können, über den Katalysator geleitet. Das Volumenverhältnis von Kohlenmonoxid zu Wasserstoff kann dabei in weiten Grenzen variiert werden. Bevorzugt sind Verhältnisse zwischen 5 : 1 und 1 : 5, insbesondere 3 : 1 bis 1 : 3. Die Reaktionstemperaturen liegen im allgemeinen zwischen 175 und 375° C, vorzugsweise zwischen 200 und 350° C.

Die Reaktionsdrucke liegen zwischen 1 und 300 bar, vorzugsweise zwischen 20 und 200 bar.

Für die Verfahrensdurchführung ist die Gasphase bevorzugt. Hierzu können die herkömmlichen Festbettreaktoren verwendet werden. Ferner sind auch Reaktoren mit bewegtem Katalysatorbett oder Wirbelbettreaktoren geeignet.

Man kann aber auch eine Umsetzung des Synthesegases in Gegenwart des festen und fein verteilten Katalysators, suspendiert in inerten Lösungsmitteln und/oder Reaktionsprodukten durchführen.

Eine besonders bevorzugte Ausführungsform der Erfindung besteht darin, die Umsetzung in einer Kreisgasapparatur in der Gasphase durchzuführen, in der nach Abtrennung der kondensierbaren Reaktionsprodukte das nicht umgesetzte Gasgemisch wieder in den Reaktor zurückgeführt wird.

Diese Verfahrensweise ist besonders wirtschaftlich und ermöglicht durch Verdünnung des Frischgases mit dem im Kreislauf zurückgeführten wasserstoffärmeren Restgas höhere Reaktionstemperaturen und damit höhere Raum-Zeit-Ausbeuten bei unveränderter Selektivität. Als Kreisgasapparaturen kommen dabei solche mit innerem oder äußerem Gasumlauf in Betracht.

Die Erfindung soll durch die folgenden Beispiele erläutert werden, die aber in keiner Weise einschränkend sein sollen.

## Beispiele

### A) Allgemeine Versuchsbeschreibung

Die Apparatur besteht aus einem beheizten Reaktionsrohr von 20 cm Länge und 14 mm innerem Durchmesser aus korrosionsbeständigem Stahl und einer zentral angebrachten Hülse zur Temperaturmessung mit einem Thermoelement.

Dieser Reaktor befindet sich in einem Heizbad aus Wood'schem Metall, in dem gleichzeitig eine Vorwärmung des Synthesegases vorgenommen wird. Die Temperatur im Reaktor sowie die des Metallbades können registrierend auf einem Schreiber verfolgt werden. Für den Fall einer Überhitzung des Katalysators ist eine $N_2$-Spülung vorhanden.

Die Reduktion des frisch in den Reaktor eingefüllten Katalysators wird mit einer $H_2/N_2$-Mischung im Verhältnis 1 : 4 bei Normaldruck und 300° C vorgenommen.

Die Synthesegasmischung ($CO : H_2 = 1 : 1$) wird einer Druckgasflasche entnommen, wobei die Menge aus dem Druckabfall, gemessen mit einem Feinmeßmanometer, ermittelt wird.

Für die Druckeinstellung und die Druckhaltung sorgt ein Druckregler. Vor diesem werden die kondensierbaren Anteile des Reaktionsgemisches in einem Abscheider verflüssigt und der GC-Analyse zugeführt.

Nach dieser Methode werden die nachstehend beschriebenen Katalysatoren geprüft. In der nachfolgenden Tabelle sind die Versuchsbedingungen sowie die Raum-Zeit-Ausbeuten an sauerstoffhaltigen $C_2$-Produkten pro Liter Katalysator und Stunde, sowie die Selektivitäten zu Ethanol zusammengestellt.

### B) Katalysatorbeschreibung

Die angegebenen Prozente sind stets Gewichtsprozente.

### Beispiel 1

Es werden 40 g eines handelsüblichen Kieselgel-Trägers mit einer spezifischen Oberfläche von 400 $m^2/g$, einem Porenvolumen von 1,2 ml/g und einem Schüttgewicht von 0,4 g/ml mit einer Lösung von 16,3 g Kobaltacetat, 3,5 g Bariumacetoaurat und 0,5 g Silberacetat in einem Gemisch von 35 ml Wasser und 5 ml Essigsäure getränkt und bei 60° C im Vakuum von 250 mbar getrocknet. Der Katalysator enthält 8,4% Co, 2,8% Au, 0,7% Ag und 1% Ba in Form der Acetate.

### Beispiel 2

35 g des in Beispiel 1 genannten Trägers werden mit einer Lösung von 16,3 g Kobaltacetat, 1,7 g Bariumacetoaurat und 2,0 g Rheniumheptoxid in 43 ml einer 7%igen Essigsäure getränkt und bei 110° C unter Normaldruck getrocknet. Der fertige Katalysator enthält 9,4% Co, 1,5% Au, 3,7% Re und 0,6% Ba.

Beispiel 3

35 g des in Beispiel 1 genannten Trägers werden mit einer Lösung von 16,3 g Kobaltacetat und 2 g Rheniumheptoxid in 43 ml einer 7%igen Essigsäure getränkt und bei 80°C in einem Stickstoffstrom getrocknet. Der fertige Katalysator enthält 9,5% Co und 3,8% Re.

Beispiel 4

Es werden 40 g eines handelsüblichen Kieselsäureträgers (250 m²/g Oberfläche, 0,8 ml/g Porenvolumen, 0,7 ml/g Schüttgewicht) mit einer Lösung von 16,3 g Kobaltacetat und 1,9 g Goldacetat in 40 ml einer 12,5%igen Essigsäure in zwei Stufen getränkt, wobei nach jeder Tränkung bis zu konstantem Gewicht getrocknet wurde. Der fertige Katalysator enthält 8,3% Co und 2% Au.

C. Versuchsergebnisse

Die Ausprüfungsergebnisse sind aus der folgenden Tabelle ersichtlich:

| Bei-spiel | Druck (bar) | Tem-peratur (°C) | Raum-geschwin-digkeit (Nl/l · h) | Raum-Zeit Ausbeute (g/l · h) | | | | | Selek-tivität*) zu Ethanol |
|---|---|---|---|---|---|---|---|---|---|
| | | | | Metha-nol | Ethanol | Propa-nole | Buta-nole | Acet-aldehyd | |
| 1 | 100 | 280 | 1200 | 7,2 | 16,5 | 4,5 | 0 | 0,05 | 60 |
| 2 | 80 | 275 | 6000 | 9,9 | 33 | 9,9 | 1,6 | 0 | 62 |
| 3 | 100 | 260 | 8050 | 8,5 | 27,5 | 6,7 | 0,7 | 0 | 65 |
| 4 | 150 | 280 | 7000 | 14,6 | 19,7 | 5,6 | 1,3 | 1,0 | 52 |
| Vergl. beisp.**) | 100 | 280 | 6500 | 0,5 | 0,04 | 0,03 | 0,01 | 0,03 | 7***) |

*) Definition der Selektivität: $\dfrac{\text{Mol CO zu Ethanol umgesetzt}}{\text{Mol CO insgesamt umgesetzt}}$

**) Vergleichsbeispiel mit reinem Kobalt; Katalysator ansonsten hergestellt wie in Beispiel 1.
***) Die übrigen Reaktionsprodukte bestehen aus gesättigten und ungesättigten Kohlenwasser-stoffen.

## Patentansprüche

1. Verfahren zur Herstellung von Ethanol aus Kohlenmonoxid und Wasserstoff an Kobalt-Katalysatoren, dadurch gekennzeichnet, daß der Katalysator außer Kobalt als Metall oder Verbindung noch mindestens eins der Elemente Gold, Silber und Rhenium als Metall oder Verbindung enthält.

2. Verfahren nach Anspruch 1, dadurch gekennzeichnet, daß die Katalysatoren auf Trägern aufgebracht sind.

## Claims

1. Process for the manufacture of ethanol from carbon monoxide and hydrogen on cobalt catalysts, characterized by using a catalyst containing, besides cobalt as metal or compound, at least one of the elements gold, silver and rhenium as metal or compound.

2. Process according to claim 1, wherein the catalysts are supported on carriers.

**Revendications**

1. Procédé de préparation de l'éthanol à partir d'oxyde de carbone et d'hydrogène sur des catalyseurs au cobalt, caractérisé en ce que le catalyseur contient, en plus du cobalt sous forme de métal ou de composé, encore au moins un des éléments or, argent et rhénium sous forme de métal ou de composé.

2. Procédé suivant la revendication 1, caractérisé en ce que les catalyseurs sont déposés sur des supports.

5